# EUROPEAN PATENT SPECIFICATION

(11) **EP 1 401 401 B1**
(45) Date of publication and mention of the grant of the patent: **30.03.2005**
(21) Application number: 02756105.9
(22) Date of filing: 24.06.2002
(51) Int. Cl.: A61K 9/14

(54) **METHOD FOR HIGH THROUGH PUT SCREENING USING A SMALL SCALE MILL OR MICROFLUIDICS**
SCREENING-VERFAHREN MIT HOHEM DURCHSATZ (HTS) UNTER VERWENDUNG VON LABORMÜHLEN ODER MICROFLUIDICS
PROCEDE POUR EFFECTUER UN CRIBLAGE A HAUT RENDEMENT AU MOYEN D'UN BROYEUR DE PETITE TAILLE OU DE PROCEDES MICROFLUIDIQUES

(30) Priority: 22.06.2001 US 299733 P; 19.10.2001 US 330093 P
(43) Date of publication of application: 31.03.2004
(73) Proprietor: Lindner, Marie, Radnor, PA 19087 (US); Elan Pharma International Limited, Shannon, County Clare (IE)
(72) Inventor: LINDNER, Marie, Radnor, PA 19087 (US); MERISKO-LIVERSIDGE, Elaine, West Chester, PA 19380 (US); CARY, Greta, Lansdale, PA 19446 (US)
(74) Representative: Khoo, Chong-Yee
(86) International application number: PCT/US2002/016589
(87) International publication number: WO 2003/000228

(56) References cited:
- US-A- 5 510 118
- US-A- 5 718 388
- US-A- 5 862 999

## Description

The invention is directed to a method of high throughput screening comprising reducing the particle size of a poorly soluble compound using a small scale mill or microfluidics to increase the solubility and/or dispersibility of the compound.

### BACKGROUND

### A. Background Relating to High Throughput Screening

Drug discovery relies on the ability to identify compounds that interact with a selected target, such as cells, an antibody, receptor, enzyme, transcription factor, or the like. Traditional drug discovery relied on collections or "libraries" obtained from proprietary databases of compounds accumulated over many years, natural products, fermentation broths, and rational drug design. Recent advances in molecular biology, chemistry, and automation have resulted in the development of rapid, HTS protocols to screen these collections. HTS and sample preparation can account for about 1% (about US$2.7 million) of developing a drug. D. McName, "Robotised assays," *Lancet, 346:* 114 (1995).

The beneficial effects of combinatorial chemistry and HTS are just beginning to be felt at the later stages of the drug pipeline. Some 40 drugs have emerged from HTS and made it to clinical trials. Directors from 50 HTS laboratories, participating in the study " High-Throughput Screening 2000: New Trends and Directions," identified 46 drug candidates that originated in their HTS laboratories, and which are being tested in humans. The backlog of new chemical entities to be screened is monumental, and the robots will continue to assay compounds, 24/7. "Screening," *Drug Discovery*/ *Technology News, 4* (2001).

Lab directors are seeking technologies to facilitate higher throughput, reduce the use of scarce compounds, cells, membranes, and reagents, and to lower reagent costs. New technologies in HTS have significantly increased throughput and reduced assay volumes. Key advances over the past few years include new fluorescence methods, detection platforms, and liquid-handling technologies. Screening 100,000 samples per day in miniaturized assay volumes will soon become routine. Hertzberg et al., "High-throughput screening: new technology for the 21st century," *Curr. Opin. Chem. Biol., 4*:445-51 (2000).

### B. Solubility of Drug Candidates

The solubility behavior of drugs remains one of the most challenging aspects in formulation development. Leuner et al., "Improving drug solubility for oral delivery using solid dispersions," *Eur. J. Pharm. Biopharm., 50*:47-60 (2000). With the advent of combinatorial chemistry and HTS, the number of poorly soluble compounds has dramatically increased. Although solid solutions have tremendous potential for improving drug solubility, forty years of research have resulted in only a few marketed products using this approach. *Id*.

The determination of solubility or dispersibility in a HTS environment is invaluable in the selection of the most promising potential drug candidates. This is because the level of permeability or solubility needed for oral absorption is related to potency. The relative importance of poor solubility and poor permeability towards the problem of poor oral absorption depends on the research approach used for lead generation. Current research approaches tend to result in a large number of poorly soluble drug candidates. For example, a "rational drug design" approach leads to time-dependent higher molecular weight, higher H-bonding properties, unchanged lipophilicity, and, hence, poorer permeability. Similarly, a HTS-based approach leads to higher molecular weight, unchanged H-bonding properties, higher lipophilicity, and, hence, poorer aqueous solubility. *Id*.

One method used to determine the solubility of potential drug candidates (usually from combinatorial chemistry) prior to HTS is based on laser nephelometry that can be supplied as dimethyl sulfoxide (DMSO) solutions in 96-well plates. Bevan et al., "A high-throughput screening method for the determination of aqueous drug solubility using laser nephelometry in microtiter plates," *Anal. Chem., 72*:1781-7 (Apr. 15, 2000). However, this method does not increase the solubility of a drug candidate, as it merely determines whether the drug is sufficiently soluble for further study.

Another method of increasing the solubility of a compound prior to HTS is to dissolve the compound in a solvent, although such a solvent can be toxic and can interfere with the activity of the compound.

Yet another method which can be used to increase solubility of a compound, but which to the best of Applicants' knowledge has not been used in conjunction with HTS, is microfluidics. While microfluidics may be employed to obtain small particles, it is not practical for larger amounts of compounds and has many inherent complications. For preparation of many screening trays for HTS, or for preparation of larger amounts of compound for use in validation of active screens, microfluidics is not appropriate. In addition, since microfluidics does not allow for stabilization of small particles, particles reduced to a nanoparticulate particle size with microfluidics must be used immediately to prevent particle size growth via agglomeration and recrystallization. In addition, compounds prepared using microfluidics cannot be scaled up for later research.

### C. Milling of Pharmaceutical Compositions

Pharmaceutical agents that exhibit poor solubility often can diminish the efficacy of a drug formulation. Improved solubility can be achieved by reducing a drug's particle size, which increases its surface area. The micronization method of grinding drug compounds to achieve a smaller particle size is well established. To the best of Applicants' knowledge, milling of pharmaceutical products has not been used in conjunction with HTS.

Conventional milling techniques, such as jet mill or rotor stator colloid mills, grind drugs into powders that have particle sizes ranging from 0.1 µm to 25 µm. Wet media mills, such as the ones described in U.S. Patent Nos. 5,797,550 issued to Woodall et al. and 4,848,676 issued to Stehr, are generally used to mill or grind relatively large quantities of materials. These rather large media mills are not generally suitable for grinding small or minute quantities, such as that required for samples to be used in or generated from HTS. U.S. Patent No. 5,593,097 issued to Corbin recognizes the need for milling small quantities, as small as 0.25 grams, to a size less than 0.5 micron to about 0.05 micron (average diameter) in about 60 minutes.

There are several research groups and companies developing and manufacturing micro-, mini-, and nanomills. For example, W.A. Bachofen, in Switzerland manufactures the DYNO®-Mill, a continuously operating bead mill with a horizontal grinder container. Bachofen make a variety of DYNO®-Mills with different specifications, such as a small laboratory model (DYNO®-Mill KDL A) which accommodates 0.15 - 0.3 liter grinding containers for discontinuous operation, and 0.3 - 0.6 liters in continuous operation. The grinding beads are spherical and have a diameter of 0.2 - 1.5 mm. The power output of the mill motor is 1.5 - 1.85 kW. One of the preferred application fields for this particular DYNO®-Mill is for mechanical cell disruption in microbiology and biochemistry. At the other end of the size and volume range is the DYNO®-Mill KD 600 that has grinders with a volume capacity of 600 liters.

A specially developed, high efficiency, bead mill for dispersion and wet grinding applications uses Bachofen's "newly developed DYNO® accelerators" (DYNO®-Mill ECM). The ECM-Pilot version accommodates 1.5 liters and has a motor output of 6.8 - 7.5 kW; the ECM-Pro model has a capacity of 18.2 liters and outputs 36 - 45 kW. In addition, the company also has an apparatus (TURBULA®) that mixes powdery substances with differing specific weights and particle sizes, and is convenient for use in the pharmaceutical industry.

Netzsch, Inc. make the LMZ Zeta System, which has a high energy, high flow, multiple pass grinding mechanism to achieve very narrow submicron size particles. Their Dynamic Cartridge Media Separator™ (DCMS) allows the use of grinding media as small as 100 µm in size. The different models can accommodate from 1.6 liters to 62 liters of suspension. One model, the MiniZeta is a high energy grinding system for small batch analysis. In this particular model, the batch size is down to 250 ml with a 4 chamber volume of 300 ml. Yet another, the Laboratory Attrition Mill is designed for very small quantities of material, wherein the grinding vessel is jacketed for cooling or heating.

MicroGrinding Systems, Inc. have made a Vibrokinetic Energy Grinding Mill, which is an "extremely fast and very energy efficient" milling machine that can be operated either wet or dry. This particular mill uses a unique tuned spring system to suspend the grinding chamber and motor energy source. This saves and reuses "rebound" energy and makes the mill cost-effective and maintenance-free, especially since the motor is the only moving part, so energy expenditure and power maintenance are minimal. Adjustable air cyclone classifiers separate product streams in the 5-10 micron range.

The mill is available in several basic models, including a Laboratory Mill "capable of producing 50 pounds per hour of fine product from a ¼" feed, and a Pilot Plant Mill which produces 250 pounds per hour of fine powder from a ¼" hard feed material. The company suggests pharmaceuticals can be ground using these apparatus.

Nanoscale Combinatorial Synthesis, Inc. (Nanosyn) is publicizing their Accelerated Nanoscale Synthesis Technology (ANST^{tm}) technology, which enables screening of compounds in miniaturized assays. Their proprietary products and services were publicized in January, 2001 when the company announced it will provide small molecule libraries to Euroscreen, a Belgium-based molecular diagnostic company.

Finally, a small scale mill exhibiting improvements over prior art technology is described in U.S. Provisional Application Serial No. 60/137,142, filed on June 1, 1999, and U.S. utility Application No. 09/583,893, filed on May 31, 2000, which arc specifically incorporated by reference. Milling of pharmaceutical or diagnostic agents to a submicron particle size is described, for example, in U.S. Patent Nos. 5,145,684 "for Surface Modified Drug Nanoparticles;" 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,494,683 for "Surface Modified Anticancer Nanoparticles;" 5,500,204 for "Nanoparticulate Diagnostic Dimers as X-Ray Contrast Agents for Blood Pool and Lymphatic System Imaging;" 5,518,187 for "Method of Grinding Pharmaceutical Substances;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabi lizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydropropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilirers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" and 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and-Particle Aggregation in Nanoparticle Compositions;" 6,270,806 for "Use of PEG-Derivatized Lipids as Surface Stabilizers for Nanoparticulate Compositions;" and 6,316,029 for "Rapidly Disintegrating Solid Oral Dosage Form;" all of which are specifically incorporated by reference. In addition, U.S. Patent Application No. 20020012675 Al, published on January 31, 2002, for "Controlled Release Nanoparticulate Compositions," describes nanoparticulate compositions, and is specifically incorporated by reference.

With the synergistic and multiplicative interactions of rational drug design, recombinant biotechnology, combinatorial chemistry, and HTS, millions of compounds are being synthesized by chemists. However, development of these candidate compounds has often been impeded, if not terminated, due to biopharmaceutic and/or pharmacokinetic constraints related to poor solubility of candidate compounds. This has resulted in delays in development time and escalation of cost in the drug research programs. Panchagnula et al., "Biopharmaceutics and pharmacokinetics in drug research," *Int. J. Pharm., 201*:131-50 (May 25, 2000).

The present invention satisfies the need in the art for rapid methods of screening compounds for acceptable bioavailability, such as pharmaceutically acceptable bioavailability, as well as increasing the solubility and/or dispersibility of candidate compounds.

### SUMMARY

The present invention is directed to a method of increasing the effectiveness of HTS, comprising reducing the particle size of a poorly soluble candidate compound to about I micron or less using a small scale mill or microfluidics.

The product produced from this process is a dispersion of a nanoparticulate candidate compound having one or more surface stabilizers adsorbed onto the surface of the compound. The reduction in particle size results in an increase in the solubility and/or dispersibility of the candidate compound, thus increasing the effectiveness of HTS conducted in conjunction with the milling or microfluidics process. The particle size reduction process, accomplished via milling or microfluidics, can be conducted before HTS to make screening compounds soluble and/or more dispersible, or after HTS to validate a poorly soluble compound determined to be active after screening. The liquid dispersion resulting from the milling or microfluidics process can be used directly in HTS.

Thus, one embodiment of the invention is directed to a method of HTS comprising milling in a small scale mill one or more poorly soluble candidate compounds to be screened to about 1 micron or less. The milling process can be performed in the presence of at least one surface stabilizer, or at least one surface stabilizer can be added to the compound dispersion following particle size reduction. Such surface stabilizers adsorb to the surface of the candidate compound, and do not chemically interact or alter the compound's properties. Following particle size reduction, the nanoparticulate compound dispersion is run through a standard HTS screen, such as an enzymatic or whole cell assay, to determine if the candidate compound has the desired activity.

Similarly, in another embodiment of the invention, one or more poorly soluble candidate compounds are subjected to microfluidization to reduce the particle size of the compounds to about 1 micron or less. The microfluidization process can be performed in the presence of at least one surface stabilizer, or at least one surface stabilizer can be added to the compound dispersion following microfluidization. Such surface stabilizers adsorb to the surface of the candidate compound, and do not chemically interact or alter the compound's properties. Following particle size reduction, the nanoparticulate compound dispersion is run through a standard HTS screen, such as an enzymatic or whole cell assay, to determine if the candidate compound has the desired activity.

Yet another embodiment of the invention is directed to a method of HTS comprising running one or more poorly soluble candidate compounds through a standard HTS screen, such as an enzymatic or whole cell assay. This is followed by reducing the particle size of the compounds identified as having the desired activity in a small scale mill, either individually or in mixtures, to about 1 micron or less to increase the solubility and/or dispersibility of the compounds to an acceptable level, such as a pharmaceutically acceptable level. The milling process can be performed in the presence of at least one surface stabilizer, or at least one surface stabilizer can be added to the compound dispersion following particle size reduction. Such surface stabilizers adsorb to the surface of the candidate compound, and do not chemically interact or alter the compound's properties.

Finally, the invention also encompasses a method of HTS comprising running one or more poorly soluble candidate compounds through a standard HTS screen, such as an enzymatic or whole cell assay. This is followed by reducing the particle size of compounds identified as having the desired activity via microfluidization, either individually or in mixtures, to about 1 micron or less to increase the solubility and/or dispersibility of the compounds to an acceptable level, such as a pharmaceutically acceptable level. The microfluidization can be performed in the presence of at least one surface stabilizer, or at least one surface stabilizer can be added to the compound dispersion following microfluidization. Such surface stabilizers adsorb to the surface of the candidate compounds, and do not chemically interact or alter the candidate compound's properties.

Both the foregoing general description and the following detailed description are exemplary and explanatory and are intended to provide further explanation of the invention as claimed. Other objects, advantages, and novel features will be readily apparent to those skilled in the art from the following detailed description of the invention.

### DETAILED DESCRIPITION OF THE INVENTION

Prior to the present invention, particle size has not been taken into account when preparing compounds for HTS screening or further drug discovery activities. Rather, compounds with poor solubility and/or dispersibility are dissolved in a solvent, which can be toxic or interfere with the activity of the compound.

An HTS method according to the invention comprises reducing the particle size of one or more poorly soluble candidate compounds to be screened, either individually or in mixtures, to about 1 micron or less using a small scale mill or microfluidics. Following particle size reduction, the nanoparticulate compound dispersion is run through a standard HTS screen, such as an enzymatic or whole cell assay, to identify compounds having a desired activity. The assays can be any known HTS assay, and can be manual or automatic.

Alternatively, the invention encompasses a method comprising running a poorly soluble candidate compound through a standard HTS screen, such as an enzymatic or whole cell assay. The assays can be any known HTS assay, and can be manual or automatic. This is followed by reducing the particle size of compounds identified as having a desired activity, either individually or in mixtures, to about 1 micron or less using a small scale mill or microfluidics. This reduction in particle size results in increasing the solubility and/or dispersibility of the compound to an acceptable level, such as a pharmaceutically acceptable level.

### Dispersion Medium

The candidate compound must be insoluble or poorly soluble in at least one liquid medium. A preferred liquid dispersion medium is water. However, the invention can be practiced with other liquid media in which a candidate compound is poorly soluble and dispersible including, for example, aqueous salt solutions, safflower oil, and solvents such as ethanol, t-butanol, hexane, and glycol. The pH of the dispersion media can be adjusted by techniques known in the art.

### "Poorly Soluble"

By "poorly soluble" it is meant that the candidate compound has a solubility in a liquid dispersion medium of less than about 10 mg/ml, and preferably of less than about 1 mg/ml. If a candidate compound is not poorly soluble, it can be conjugated to a salt or other substance to render the compound poorly soluble. Thus, all candidate compounds having, for example, therapeutic, cosmetic, diagnostic, or bioengineering uses are presumed suitable for the invention. The term "candidate compound" is not limited to a substance having pharmaceutical activity, as the invention is intended to encompass any and all poorly soluble compounds or compounds which can be made poorly soluble, and which has a desired activity, for example, compounds useful in pharmaceuticals, cosmetics, diagnostics, bioengineering, and agriculture, such as pesticides, ferilizers, insecticides, and herbicides.

For example, if the candidate compound is soluble in the liquid dispersion medium, the compound can be conjugated to other molecules or moieties to render the compound poorly soluble prior to milling. Compounds can be conjugated to, for example, hydrophobic molecules, molecules with amphipathic properties, lipid molecules, phospholipid molecules, fats, prenyl groups, or palmitoyl groups to render the candidate compound less soluble or poorly soluble prior to milling or microfluidization. Such conjugation can be through direct conjugation to specific sites on the compound, to the N-terminal or C-terminal residue of the compound via intermediate spacer molecules which can be attached to one or more sites on the compound, and/or through internal side chains on the compound.

Furthermore, a compound can be rendered less soluble by addition of amino acid residues either during the chemical synthesis or the biological expression of the compound, in particular, amino acid residues or derivatives with hydrophobic properties. Such residues or motifs can be separated from the compound by hydrolysable linkers or linkers which can be cleaved *in vivo*, for example, by specified enzymes or csterases.

In addition, the candidate compounds can be conjugated to pharmaceutically acceptable salts to render the compounds poorly soluble. Furthermore, the compounds can be rendered poorly soluble by adjusting the pH of the dispersion medium.

### Exemplary Milling Methods

One milling method according to the present invention comprises providing a dispersion of one or more poorly-soluble candidate compounds to be milled and attrition milling media. Preferred attrition media has a particle size of 500 microns or less.

For an exemplary milling machine, the dispersion is inserted into a vessel, such as a cylindrical or other shaped vessel, and an agitator and a coupling that closes the vessel are provided. The coupling has an opening through which a portion of the agitator extends, and the agitator comprises a cylindrical rotor and a shaft extending therefrom, wherein the cylindrical rotor is dimensioned such that an outer periphery is minimal, for example, no greater than 3 mm away from an inner surface of the wall, although other size ranges can be employed in the invention and the exemplary amount is not intended to be limiting. An agitator is inserted into the vessel and the coupling is sealed or closed, wherein the amount of dispersion inserted into the vessel is such that the dispersion eliminates substantially all of the air in the vessel when the agitator is fully inserted into the vessel. The agitator is then rotated for a predetermined period. One or more surface stabilizers for the candidate compound(s) are added to the dispersion either before or after milling.

Another method according to the present invention comprises providing a dispersion comprising one or more poorly soluble candidate compounds to be milled and attrition milling media. Preferred attrition media has a particle size of 500 microns or less. An agitator having a cylindrical rotor and shaft extending therefrom is provided, the agitator is inserted in a horizontally oriented vessel, and the vessel is sealed. The rotor is dimensioned to provide a minimal gap, for example, no greater than 3 mm between an outer surface of the rotor and an inner surface of the vessel, although other size ranges can be employed in the invention and the exemplary amount is not intended to be limiting. At least one port through the vessel is provided, and the port is maintained at the highest point of the horizontally oriented vessel. The vessel is filled with the compound dispersion until the dispersion drives out substantially all of the air in the vessel. Finally, the agitator is rotated for a predetermined period. One or more surface stabilizers for the candidate compound(s) are added to the dispersion either before or after milling.

Because virtually all of the air can be displaced in the vertically and horizontally oriented mills, vortexing and contamination problems are minimized or avoided. Thus, the milling process according to the present invention can prevent the dispersion formulation from foaming.

### Exemplary Microfluidization Method

U.S. Patent No. 5,510,118, for "Method for Preparing Therapeutic Compositions Containing Nanoparticles," describes an exemplary method of making sub-micron sized poorly soluble compounds using microfluidization. This patent is specifically incorporated by reference.

### Advantages of the HTS Method of the Invention

One advantage of the HTS methods of the invention, when the dispersion medium is water, is that for whole cell HTS screens, the milled or microfluidized aqueous compound dispersion of the invention is non-toxic, as water is non-toxic to cells. This is in contrast to prior art methods, in which poorly soluble compounds were solubilized in solvents. As a result, cellular activity is more clearly observed for the dispersions of the invention, since there is no solvent-induced cell toxicity.

Another advantage of the methods of the invention is that the milled or microfluidized dispersions can be used directly in HTS by aliquoting the correct concentration into wells to run through standard HTS screens. Additionally, the concentration can vary between different wells of the HTS assay. Milled and microfluidized compound dispersions can also be used in other enzymatic or cellular tests of activity and toxicity. Again, an advantage of the present invention is that no toxic solvent is present in the milled dispersion. In addition, the compound requires very little reformulation work for clinical studies.

Nanoparticulate dispersions prepared according to the invention are stable for extensive periods of time, i.e., for a year or more. Thus, the HTS method of the invention does not require immediately screening a compound following milling or microfluidization. Moreover, compounds prepared according to the invention can be readily scaled up for manufacturing.

The time required to prepare a milled micro- or nanoparticulate suspension from a given amount of starting material is on average about one hour or less. Thus 3-4 samples, or more, can be comfortably milled within a working day with one small scale mill, including preparation time, milling, harvesting, and particle sizing of the milled dispersion. The time limiting factor is preparation and analyzation of the resulting sample; with pre-prepped samples, about 6-8 compounds or more could be milled per day in each small scale mill.

In general, the time required for the particle size reduction process is selected from the group consisting of about one hour or less, about 45 minutes or less, about 40 minutes or less, about 35 minutes or less, about 30 minutes or less, about 25 minutes or less, about 20 minutes or less, about 15 minutes or less, about 10 minutes or less, and about 5 minutes or less.

### Attrition Media

The attrition media used in a small scale mill can be a polymeric type, such as formed of polystyrene or cross-linked polystyrene having a nominal diameter of no greater than 500 microns. Other particle sizes of useful milling media include 200 microns and 50 microns, and a mixtures of sizes ranging between about 50 and about 500 microns.

U.S. Patent Nos. 5,518,187, 5,718,388, and 5,862,999 disclose milling pharmaceutical products using polymeric milling media. These patents further disclose dispersion formulations for a wet media milling. The disclosures of these patents are specifically incorporated by reference.

### Surface Stabilizers

The one or more surface stabilizers are adsorbed on the surface of the candidate compound in an amount sufficient to maintain the candidate compound at an effective average particle size of less than about 1 micron, or other desired particle size.

The relative amount of the candidate compound and surface stabilizer can vary widely and the optimal amount of the surface stabilizer can depend, for example, upon the particular candidate compound and surface stabilizer selected, the critical micelle concentration of the surface stabilizer if it forms micelles, *etc*.

The at least one surface stabilizer is present in the liquid dispersion medium in an amount selected from the group consisting of from about 0.01% to about 90%, about 1% to about 90%, and about 5% to about 90%, by weight, based on the total dry weight of the candidate compound and surface stabilizer. The one or more surface stabilizers can be added to the liquid dispersion medium either before or after size reduction of the one or more candidate compounds.

Useful surface stabilizers, which are known in the art and described in U.S. Patent No. 5,145,684, specifically incorporated by reference, are believed to include those which physically adhere to the surface of the candidate compound but do not chemically bond to or interact with the compound. Furthermore, the individually adsorbed molecules of the surface stabilizer are essentially free of intermolecular cross-linkages. Two or more surface stabilizers can be employed in the methods of the invention.

Suitable surface stabilizers can preferably be selected from known organic and inorganic pharmaceutical excipients. Such excipients include various polymers, low molecular weight oligomers, natural products, and surfactants. Preferred surface stabilizers include nonionic and ionic surfactants.

Representative examples of surface stabilizers include gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g*., macrogol ethers such as cetomacrogol 1000), polyoxyethylene castor oil derivatives, polyoxyethylene sorbitan fatty acid esters (*e.g*., the commercially available Tweens® such as *e.g.,* Tween 20® and Tween 80® (ICI Speciality Chemicals)); polyethylene glycols (*e*.*g*., Carbowaxs 3550® and 934® (Union Carbide)), polyoxyethylene stearates, colloidal silicon dioxide, phosphates, sodium dodecylsulfate, carboxymethylcellulose calcium, carboxymethylcellulose sodium, methylcellulose, hydroxyethylcellulose, hydroxypropylcellulose, hydroxypropylmethyl-cellulose phthalate, noncrystalline cellulose, magnesium aluminium silicate, triethanolamine, polyvinyl alcohol (PVA), polyvinylpyrrolidone (PVP), 4-(1,1,3,3-tetramethylbutyl)-phenol polymer with ethylene oxide and formaldehyde (also known as tyloxapol, superione, and triton), poloxamers (*e*.*g*., Pluronics F68® and F108®, which are block copolymers of ethylene oxide and propylene oxide); poloxamines (*e.g*., Tetronic 908®, also known as Poloxamine 908®, which is a tetrafunetional block copolymer derived from sequential addition of propylene oxide and ethylene oxide to ethylenediamine (BASF Wyandotte Corporation, Parsippany, N.J.)); Tetronic 1508® (T-1508) (BASF Wyandotte Corporation), dialkylesters of sodium sulfosuccinic acid (*e*.*g*., Aerosol OT®, which is a dioctyl ester of sodium sulfosuccinic acid (American Cyanamid)); Duponol P®, which is a sodium lauryl sulfate (DuPont); Tritons X-200®, which is an alkyl aryl polyether sulfonate (Rohm and Haas); Crodestas F-110®, which is a mixture of sucrose stearate and sucrose distearate (Croda Inc.); p-isononylphenoxypoly-(glycidol), also known as Olin-IOG® or Surfactant 10-G® (Olin Chemicals, Stamford, CT); Crodestas SL-40® (Croda, Inc.); and SA9OHCO, which is C₁₈H₃₇CH₂C(O)N(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, and random copolymers of vinyl acetate and vinyl pyrrolidone (*i*.*e*., Plasdone® S630), and the like.

Most of these surface stabilizers are known pharmaceutical excipients and are described in detail in the *Handbook of Pharmaceutical Excipients*, published jointly by the American Pharmaceutical Association and The Pharmaceutical Society of Great Britain (The Pharmaceutical Press, 1995), specifically incorporated by reference. The surface stabilizers are commercially available and/or can be prepared by techniques known in the art.

### Candidate Compound/Surface Stabilizer Particle Size

The compound of the invention is reduced to an effective average particle size of less than about 1 micron. The compound can also be reduced to an effective average particle size of less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, and less than about 50 nm. Such small effective average particle sizes can generally not be obtained using conventional mills.

As used herein, particle size is determined based on the weight average particle size as measured by conventional particle size measuring techniques well known to those skilled in the art. Such techniques include, for example, sedimentation field flow fractionation, photon correlation spectroscopy, light scattering, and disk centrifugation.

By "an effective average particle size of less than about I micron" it is meant that at least 50% of the candidate compound particles have an average particle size of less than about I micron when measured by the above techniques. Preferably, at least at least 60%, 70%, 80%, 90%, or 95% of the candidate compound particles are reduced to a particle size less than the effective average particle size, *i.e*., less than about I micron, less than about 900 nm, less than about 800 nm.

### Concentration/Quantity of Candidate Compound; Dispersion Volume Required

A small quantity of a candidate compound can be processed using the milling and microfluidization methods of the invention. For example, 100 mg of a candidate compound (a 2% dispersion) can be used, and smaller amounts can also be used. Higher concentrations of candidate compound, at for example, 5% up to about 50%, can also be milled or microfluidized. 100 mg (2% dispersion) generally corresponds to 4-6 ml of total dispersion volume. The amount of candidate compound can be drug dependent; for milling and microfluidization, the dispersion must be fluid and non-viscous.

In the methods of the invention, the candidate compound is present in a concentration selected from the group consisting of less than about 50%, less than about 40%, less than about 30%, less than about 25%, less than about 20%, less than about 15%, less than about 10%, less than about 5%, less than about 4%, less than about 3%, less than about 2%, less than about 1%, less than about 0.5%, less than about 0.1%, less than about 0.01%, and less than about 0.001%.

Alternatively, the candidate compound is present in an amount selected from the group consisting of from about 90% to about 0.001%, from about 90% to about 0.1%, and from about 60% to about 5%, by weight, based on the total dry weight of the candidate compound and surface stabilizer.

The quantity of candidate compound required for the particle size reduction process is selected from the group consisting of less than about 100 mg, less than about 90 mg, less than about 80 mg, less than about 70 mg, less than about 60 mg, less than about 50 mg, less than about 40 mg, less than about 30 mg, less than about 25 mg, less than about 20 mg, less than about 15 mg, less than about 10 mg, less than about 5 mg, less than about 4 mg, less than about 3 mg, less than about 2 mg less than about 1 mg, and less than about 0.5 mg.

In addition, the total dispersion volume required for the particle size reduction process is selected from the group consisting of less than about 15 mL, less than about 10 mL, less than about 9 mL, less than about 8 mL, less than about 7 mL, less than about 6 mL, less than about 5 mL, less than about 4 mL, less than about 3 mL, and less than about 2 mL.

### Small Scale Mill Structure

A small-scale mill according to the present invention is designed to mill relatively small amounts of dispersion to a size ranging from microns-to nanometers in a relatively short time, *i*.*e*., one or more hours or less, using attrition milling media, such as polymeric media type, *e.g.,* cross linked polystyrene media, having a particle size-of about 500 microns (0.5 mm) or less to about 50 microns, or mixtures of the sizes ranging between 500 and 50 microns.

A preferred small scale mill useful in the invention is described in WO 00/72973 for "Small Scale High Energy Mill," published on December 7, 2000. Such a mill has few moving parts, has easy set up and clean up as it can be quickly dismantled, and it has a small footprint, which is critical in a laboratory setting. This is in contrast, for example, to Dymomill® mills which have many moving parts.

A preferred small scale mill is a table-top unit with a small foot-print, and several small scale mills can be used simultaneously to increase the screening of compounds.

In addition, a preferred small scale mill uses a water cooling system to allow for effective removal of excess heat generated during milling. In one embodiment, the cooling system can comprise a water jacket; in another embodiment, the mill chamber is double-walled to allow for circulation of coolant. In addition, three or more mills can be set up with a single cooling system. The presence of such a cooling systems allows for milling at higher speeds. Also, preferably the milling speed of the small scale mill can be varied. The combined effect of cooling and variability of milling speed makes the small scale mill an effective tool for temperature and/or milling energy sensitive compounds.

Yet another advantage of the preferred small scale mill is that rotors can be changed. Smooth shafts produce shear milling forces, while pegged shafts produce shear and impact forces. A pegged shaft is useful for a compound which is difficult to mill. Moreover, with the same milling head, different chamber sizes can be used, *i*.*e*., chamber sizes of 10, 18, and 26 mls (in general, the dispersion size is about ½ of the chamber size). This interchangability of parts is a significant improvement over prior art milling technologies.

The rotor can be cylindrical, and can have tapered end surfaces. In one embodiment, the rotor is dimensioned so that its outer periphery is spaced no larger than 3 mm away from an inner surface of the vessel, particularly when the dispersion contains attrition media having a particle size of 500 microns or less. The spacing or the gap is preferably no larger than I mm, particularly when the dispersion contains attrition media having a particle size of 200 microns or less.

The vessel size can vary for milling small amounts of dispersion. Although the present invention is not limited to particular sizes, in a preferred embodiment the inner diameter of the vessel is between 5/8 inch to 4 inches. By way of example only, a milling chamber and a cylindrical rotor can have the dimensions specified in Tables 1 and 2.

**TABLE 1**

| **(STRAIGHT ROTORS)** | | | |
|---|---|---|---|
| **CYLINDRICAL VESSEL Size** | **#1** | **#2** | **#3** |
| Volume Vessel (in³) | 1.658 | 3.090 | 4.963 |
| Volume Rotor (in³) | 0.899 | 1.866 | 3.156 |
| Volume Shaft (in³) | 0.036 | 0.036 | 0.036 |
| Working Volume (in³) | 0.723 | 1.187 | 1.770 |
| | 11.855 ml | 19.458 ml | 29.012 ml |
| Typical Dispersion Volume @ 50% media charge | 8.299 ml | 13.621 ml | 20.309 ml |
| Typical Dispersion Volume @ 90% media charge | 5.453 ml | 8.95 ml | 13.346 ml |

| **TABLE 2 (TAPERED ROTORS)** | | | |
|---|---|---|---|
| VESSEL Size | #1 | #2 | #3 |
| | | | |
| Volume Vessel (in³) | 1.754 | 3.268 | 5.250 |
| Volume Rotor Body (in³) | 0.899 | 1.726 | 2.919 |
| Volume Upper Cone (in³) | 0.040 | 0.128 | 0.196 |
| Volume Lower Cone (in³) | 0.040 | 0.080 | 0.122 |
| Volume Shaft (in³) | 0.026 | 0.026 | 0.026 |
| Volume Complete Rotor (in³) | 0.979 | 1.934 | 3.237 |
| | | | |
| Working Volume (in³) | 0.749 | 1.308 | 1.986 |
| | 12.274 ml | 21.429 ml | 32.548 ml |
| | | | |
| Typical Dispersion Volume @ 50% media charge | 8.592 ml | 15.001 ml | 22.784 ml |
| | | | |
| Typical Dispersion Volume @ 90% media charge | 5.646 ml | 9.858 ml | 14.972 ml |

The following examples are given to illustrate the present invention. It should be understood, however, that the invention is not to be limited to the specific conditions or details described in these examples. Throughout the specification, any and all references to a publicly available documents are specifically incorporated into this patent application by reference.

### Example 1

The purpose of this example was to demonstrate the effectiveness of using high energy milling technology when formulating milligram quantities of poorly water soluble compounds for pre-clinical in vivo studies.

**Methods.** Small volume high-energy media mills capable of processing <100 mg of drug were tested for efficiency and performance using naproxen as the poorly water soluble drug candidate. A statistical design study was performed to test the robustness of the process and identify formulation parameters required to generate nanoparticle suspensions having a mean particle size of less than 200 nm.

For processing, naproxen was milled in aqueous based stabilizer solutions at various concentrations for 15 min. to 60 min. at 10°C. The quality of the dispersion was evaluated using microscopy and laser light diffraction.

Naproxen has a molecular weight of 230.3 g, and a solubility in water of 16 µg/mL at pH 2 and 3.2 mg/mL at pH 7.5. The drug was milled at a low pH. The milling conditions were as follows: 3000-6000 rpm; 0.5% - 2% drug loading; 15 - 60 min. milling time; 60% - 90% media load; and 4 different mills were used (all were NanoMills™, manufactured by Elan Drug Delivery, Inc.).

A Greco Latin Square Design format was used and the experiment order was randomized. The table below shows the experiments performed:

**Table 3**

| **Experiment (rpm)** | **Mill Speed** | **Media Load (%)** | **Milling Time (min.)** | **Drug Conc. (%)** | **Mill #** |
|---|---|---|---|---|---|
| 1 | 3000 | 70 | 30 | 1 | 2 |
| 2 | 3000 | 60 | 15 | 0.5 | 1 |
| 3 | 4000 | 70 | 15 | 2 | 3 |
| 4 | 3000 | 90 | 60 | 2 | 4 |
| 5 | 3000 | 80 | 45 | 1.5 | 3 |
| 6 | 4000 | 80 | 60 | 0.5 | 2 |
| 7 | 4000 | 60 | 30 | 1.5 | 4 |
| 8 | 6000 | 80 | 30 | 2 | 1 |
| 9 | 5000 | 80 | 15 | 1 | 4 |
| 10 | 4000 | 90 | 45 | 1 | 1 |
| 11 | 6000 | 90 | 15 | 1.5 | 2 |
| 12 | 5000 | 90 | 30 | 0.5 | 3 |
| 13 | 5000 | 70 | 60 | 1.5 | 1 |
| 14 | 5000 | 60 | 45 | 2 | 2 |
| 15 | 6000 | 60 | 60 | 1 | 3 |
| 16 | 6000 | 70 | 45 | 0.5 | 4 |

The important parameters for milling in a small scale mill are mill speed (rpm), percent media load, drug concentration, the interaction between mill speed and media load, the interaction between milling time and drug concentration, and the interaction between mill speed, percent media load, and drug concentration.

**Results.** The following preferred formulation and milling parameters were identified for obtaining a composition having a particle size of less than about 200 nm.

Mill Speed = 4300 rpm; minimum milling time = 15 min.; maximum milling time = 60 min.; minimum drug concentration = 0.5%; maximum drug concentration = 20%; and final yield was about 75%.

The study shows that a stable nanoparticle formulation of naproxen can be generated with < 50 mg of drug in 15 min. using a small-volume high energy mill. The nanoparticle suspensions were homogeneous as monitored by optical microscopy and exhibited a unimodal particle size distribution profile with a mean diameter of less than 200 nm. Approximately 90% of the drug was harvested after processing. Physical stability of the harvested formulations was acceptable after storage under refrigeration for at least two weeks.

**Conclusions.** Small-scale high energy wet-milling technology can be successfully utilized to generate stable formulations of poorly water soluble drugs in less than 15 min. with as little as 25 mg of drug. This approach provides an alternate method for effectively formulating poorly water soluble drugs that does not involve the use of solvents and is ideal for preclinical bioavailability and toxicology studies.

### Example 2

The purpose of this example was to demonstrate the reproducability of the small scale milling process described in Example 1 using naproxen and several different new chemical entities.

Naproxen and five different poorly soluble new chemical entities having various chemistries, various mechanisms of action, and targeting different medical indications were milled as in Example 1. The results of the tests are shown below.

**Table 4**

| **Drug** | **Amount Milled (mg)** | **Milling Time (min.)** | **Particle Size (nm)** |
|---|---|---|---|
| Naproxen | 50 | 15 | 159 |
| Naproxen | 200 | 60 | 147 |
| Compound 1 | 200 | 60 | 93 |
| Compound 2 | 200 | 60 | 166 |
| Compound 3 | 200 | 60 | 162 |
| Compound 4 | 200 | 60 | 188 |
| Compound 5 | 200 | 60 | 168 |

The results demonstrate that the milling method is applicable to a wide variety of compounds, and is not limited by the chemical entity to be milled.

### Example 3

The purpose of this example was to demonstrate the effectiveness of scale-up of milling experiments conducted in a small scale mill to a large batch size milling process.

Naproxen was milled in five different media mill sizes: (1) 25 mg - 1 g; (2) 4 g - 2 kg; (3) 1 - 10 kg; (4) 10 - 100 kg; and (5) 100 - 1000 kg. The results of the experiment are shown below. The value for D50 is the particle size below which 50% of the naproxen particles fall. Similarly, D90 is the particle size below which 90% of the naproxen particles fall.

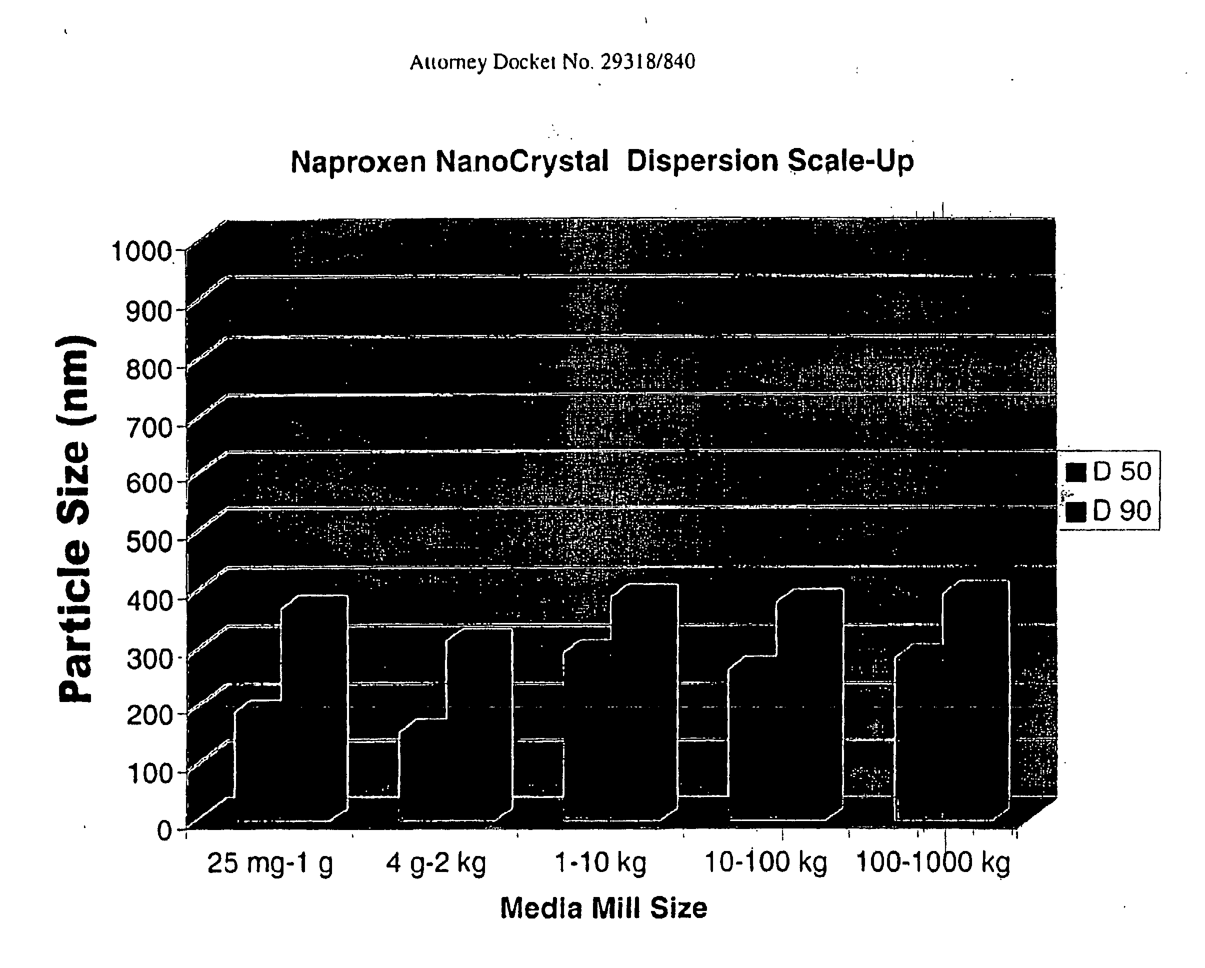

The results given above demonstrate the consistency in particle size from milling in small quantities up to larger manufacturing scale quantities, particularly for D90 *(i.e.,* the particle size below which 90% of the particles of a composition fall). This is significant, as high throughput screening methods to identify suitable candidates for preclinical bioavailability and toxicoligy studies are significantly more useful if the screening methods used can be easily scaled up for manufacturing.

### Example 4

The purpose of this example was to demonstrate the effectiveness of milling an extremely small quantity of active agent in a small scale mill.

About 15 mg of Compound X (0.5% drug) was combined with 0.25% Pluronic® F108 and 0.25% Na Deoxycholate for 60 minutes in a NanoMill™ (Elan Drug Delivery, Inc.). 6 mL of 0.8 mm YTZ grinding media (Yittria treated Zirconia; Tosoh Corporation) was used in the milling process.

The resultant formulation was well-dispersed and had an average particle size of about 300 nm , based on light microscopy analysis.

### Example 5

The purpose of this example was to demonstrate successful small scale milling of very small quantities of drug.

Naproxen, polyvinylpyrrolidone (PVP) K29/32, and sodium lauryl sulfate (SLS) were combined in a ratio of 5:2:0.05, with naproxen present at 0.0625%. The mixture was milled in a NanoMill™ (Elan Drug Delivery, Inc.). Using a media load of 100% at the maximum rpm of 6000 maximized the energy input.

Further calculations were based on a bulk density of 0.61g/ml for PolyMill™ 500 µ media (Dow Chemical) and a void volume of 40%. Since the amounts were low and mixing difficult, PVP and SLS were prepared as 20% and 5% stock solutions, respectively. The calculations for milling in a 10 mL, 18 mL, and 26 mL milling chambers are summarized in the following chart:

**Table 5**

| | **10 mL chamber** | **18 mL chamber** | **26 mL chamber** |
|---|---|---|---|
| **RPM** | 6000 | 6000 | 6000 |
| **Media volume** | 10 ml | 18 ml | 26 ml |
| **Media quantity; PolyMill™-500µ** | 6.1 g | 11.0 g | 15.9 g |
| **Dispersion volume** | 4.0 ml | 7.2 ml | 10.4 ml |
| **Naproxen** | 25 mg | 45 mg | 65 mg |
| **PVP 29/32; 20%** | 50 mg | 90 mg | 130 mg |
| **SLS; 5%** | 5 mg | 9 mg | 13 mg |
| **Water For Injection** | 3.92 g | 7.06 g | 10.27 g |

### 10 ml chamber:

Analysis of the resultant particle size of the naproxen dispersion using a Horiba LA-910 Laser Scattering Particle Size Distribution Analyzer (Horiba Instruments, Irvine, CA) indicated that although a stable dispersion was formed, milling was not complete. A large aggregate peak of larger material was present in the particle sizing results. This peak decreased slightly over time, but was still present after 1 hr. The bimodal particle size peak had a mean of 1496 nm and a median of 354 nm. The median is therefore representative of the primary peak; the mean is higher due to the large aggregate of unmilled material still present in the sample.

### 18 ml chamber:

Complete milling of the naproxen dispersion was observed. Particle size analysis using a Horiba LA-910 Laser Scattering Particle Size Distribution Analyzer showed a particle size of essentially one single peak. The single peak mean particle size was 314 nm.

### 26 ml chamber:

Complete milling of the naproxen dispersion was observed. Particle size analysis using a Horiba LA-910 Laser Scattering Particle Size Distribution Analyzer showed a particle size of essentially one single peak, although the peak was much broader than the 18 mL sample. -Narrower peaks correspond to a narrower particle size distribution. The single peak mean particle size was 630 nm.

### Example 6

The purpose of this example was to mill a constant amount of active agent in different size milling chambers to determine the effect on resultant active agent particle size. The effect of changing only the size of the milling chamber was to reduce the percentage of drug present.

25 mg samples of naproxen were milled in 10 mL, 18 mL, and 26 mL chambers of a NanoMill™. Naproxen, polyvinylpyrrolidone (PVP) K29/32, and sodium lauryl sulfate (SLS) were combined in a ratio of 5:2:0.05. Analysis of resultant particle size was via photomicrography.

### 10 ml chamber:

A reasonable naproxen dispersion was formed after 15 min. Although a large population of larger and unmilled particles was clearly present, this was attributed to the chamber and rotor configuration and not the formulation. Later samples at 30 and 60 min. showed no improvement and were perhaps more heterogeneous.

### 18 ml chamber:

A reasonable naproxen dispersion was formed in 15 minutes, although the dispersion was more heterogeneous than that of the 10 ml chamber. The 30 min. sample was clearly over-milled and aggregated.

### 26 ml chamber:

Since the higher energy and/or greater time seemed to cause the sample to overmill, this sample was viewed after 5 min. and showed a dispersion with excellent homogeneity, although larger particles were also present. After 15 min., the sample was clearly degenerating.

Examples 5 and 6 demonstrate-that at extremely low levels of active agent, very little energy is required to achieve a "reasonable" dispersion that indicates whether or not a Formulation screened was a viable candidate. In sum, in screening active agents to determine potential usefulness, use of a 10 mL chamber sampled at a 5 min. time point should be sufficient. Such a method has the advantage of avoiding over-milling of the sample if the dispersion is checked at later time points after continued milling.

### Example 7

The purpose of this example is to demonstrate successful milling of an extremely small quantity of an active agent.

5 mg of naproxen, and PVP and SLS, were milled in a 10 mL chamber of a NanoMill™. Naproxen, PVP K29/32, and SLS were combined in a ratio of 5:2:0.05.

It was found that 5 mg of active agent, such as naproxen, in a 10 ml chamber was sufficient to form a dispersion that could be shown under photomicrography to be a well dispersed nano-suspension. Although larger particles were also present, this was attributed to the parameters used and the chamber and rotor configuration and not the formulation.

Thus, minute quantities, such as 5 mg, of active agent can be milled to determine the potential suitability of the composition for formulating in a nanoparticulate composition to increase bioavailability of the active agent.

### Example 8

The purpose of this example is to demonstrate successful milling of an extremely small quantity of an active agent. A compound, Photogen (WIN 67722; 6-ethoxy-6-oxohexyl-3,5-bis(acetamido)-2,4,6-triiodobenzoate), was screened using the technique of Example 7. Photogen is an iodinated imaging agent.

A mixture of 15% Photogen and 3% Pluronic® F-108 was adjusted to a ratio of 5:2 and decreased to 10 mg active agent. The composition was then milled in a 10 mL chamber of a NanoMill™. Although the resultant particle size was larger than -naproxen, well-dispersed particles were seen in 5 min. and smaller particles were formed in 15 and 30 min. The resultant particle size was predominantly sub-micron.

This example further demonstrates the usefulness of screening active agents in the drug discovery stage, when quantities of active agent may be limited, using a small scale mill or microfluidics, requiring small or minute quantities of active agent to produce nanoparticulate dispersions.

## Claims

1. A high throughput screening method comprising:
(a) reducing the particle size of one or more candidate compounds in a small scale mill in the presence of attrition milling media, wherein:
(1) the one or more candidate compounds are milled in a liquid dispersion medium in which the candidate compounds are poorly soluble;
(2) the milled one or more candidate compounds have an effective average particle size of less than about 1 micron, and
(3) at least one surface stabilizer is added to the liquid dispersion medium, either before or after particle size reduction, in an amount sufficient to maintain the effective average particle size of the one or more candidate compounds, following particle size reduction, at less than about 1 micron; and
(b) screening the one or more nanoparticulate candidate compounds in a conventional high throughput screening assay to determine if the one or more compounds have a desired activity.

2. A high throughput screening method comprising:
(a) screening one or more candidate compounds in a conventional high throughput screening assay to determine if the one or more compounds have a desired activity; and
(b) reducing the particle size of the one or more candidate compounds in a small scale mill in the presence of attrition milling media, wherein:
(1) the one or more candidate compounds are milled in a liquid dispersion medium in which the candidate compounds are poorly soluble;
(2) the milled one or more compounds have an effective average particle size of less than about 1 micron, and
(3) at least one surface stabilizer is added to the liquid dispersion medium, either before or after particle size reduction, in an amount sufficient to maintain the effective average particle size of the one or more candidate compounds, following particle size reduction, at less than about 1 micron, and
(4) determining if the one or more compounds have acceptable solubility and/or dispersibility.

3. A high throughput screening method comprising:
(a) reducing the particle size of one or more candidate compounds using homogenization wherein:
(1) the one or more candidate compounds are reduced in size in a liquid dispersion medium in which the candidate compounds are poorly soluble;
(2) the homogenized one or more candidate compounds have an effective average particle size of less than about 1 micron, and
(3) at least one surface stabilizer is added to the liquid dispersion medium, either before or after particle size reduction, in an amount sufficient to maintain the effective average particle size of the one or more candidate compounds, following particle size reduction, at less than about 1 micron; and
(b) screening the one or more nanoparticulate candidate compounds in a conventional high throughput screening assay to determine if the one or more compounds have a desired activity.

4. A high throughput screening method comprising:
(a) screening one or more candidate compounds in a conventional high throughput screening assay to determine if the one or more compounds have a desired activity; and
(b) reducing the particle size of the one or more candidate compounds in a small scale mill in the presence of attrition milling media, wherein:
(1) the one or more candidate compounds are milled in a liquid dispersion medium in which the candidate compounds are poorly soluble;
(2) the milled one or more compounds have an effective average particle size of less than about 1 micron, and
(3) at least one surface stabilizer is added to the liquid dispersion medium, either before or after particle size reduction, in an amount sufficient to maintain the effective average particle size of the one or more candidate compounds, following particle size reduction, at less than about 1 micron, and
(4) determining if the one or more compounds have acceptable solubility and/or dispersibility.

5. A method according to Claim 3 or 4, in which the one or more candidate compounds are homogenized in the presence of attrition media.

6. A method according to Claim 1, 3 or 4, or Claim 5 as dependent thereon, in which the dispersion of nanoparticulate candidate compounds from step (a) is used directly in the high throughput screening assay of step (b).

7. A method according to Claim 1 or 3, or Claim 5 or 6 as dependent thereon, in which a mixture of two or more candidate compounds is reduced in size in step (a).

8. A method according to Claim 1 or 3, or Claim 5, 6 or 7 as dependent thereon, in which a mixture of two or more candidate compounds is screened in step (b).

9. A method according to Claim 2 or 4, or Claim 5 or 6 as dependent thereon, in which a mixture of two or more candidate compounds is reduced in size in step (b).

10. A method according to Claim 2 or 4, or Claim 5, 6 or 7 as dependent thereon, in which a mixture of two or more candidate compounds is screened in step (a).

11. A method according to any preceding claim, in which the attrition media or the attrition milling media is polymeric.

12. A method according to any preceding claim, in which the attrition media or the attrition milling media has a particle size selected from the group consisting of about 500 microns or less, about 200 microns or less, about 50 microns or less, and mixtures thereof.

13. A method according to any preceding claim, in which the high throughput screening assay is an enzymatic or whole cell assay.

14. A method according to any preceding claim, in which the dispersion medium is selected from the group consisting of water, aqueous salt solutions, safflower oil, ethanol, t-butanol, 5 hexane, and glycol.

15. A method according to any preceding claim, in which the high throughput screening assay is manual or automatic.

16. A method according to any preceding claim, in which the candidate compound has a solubility in the liquid dispersion medium of less than about 10 mg/ml.

17. A method according to any preceding claim, in which the candidate compound has a solubility in the liquid dispersion medium of less than about 1 mg/ml.

18. A method according to any preceding claim, in which the candidate compound is conjugated to a salt or other substance to render the candidate compound poorly soluble.

19. A method according to any preceding claim, in which the candidate compound is conjugated to a substance selected from the group consisting of hydrophobic molecules, molecules with amphipathic properties, lipid molecules, phospholipid molecules, fats, prenyl groups, and palmitoyl groups.

20. A method according to Claim 18 or 19, wherein such conjugation is accomplished by a method selected from the group consisting of direct conjugation to specific sites on the compound, conjugation to the N-terminal or C-terminal residue of the compound via intermediate spacer molecules, and conjugation through internal side chains on the compound.

21. A method according to any preceding claim, in which the candidate compound is rendered poorly soluble by the addition of amino acid residues either during the chemical synthesis or the biological expression of the compound.

22. A method according to any preceding claim, in which the candidate compound is rendered poorly soluble by adjusting the pH of the dispersion medium.

23. A method according to any preceding claim, in which the candidate compound is selected from the group consisting of a therapeutic agent, a cosmetic, a diagnostic agent, an agent useful in bioengineering, and an agricultural agent.

24. A method according to any preceding claim, in which the candidate compound is an agricultural agent selected from the group consisting of a pesticide, a fertilizer, an insecticide, and a herbicide.

25. A method according to any preceding claim, in which the time between conducting step (a) and conducting step (b) extends for up to one year.

26. A method according to any preceding claim, in which the candidate compound is present in a concentration selected from the group consisting of less than about 50%, less than about 40%, less than about 30%, less than about 25%, less than about 20%, less than about 15%, less than about 10%, less than about 5%, less than about 4%, less than about 3%, less than about 2%, less than about 1%, less than about 0.5%, less than about 0.1%, less than about 0.01 %, and less than about 0.001%.

27. A method according to any preceding claim, in which the candidate compound is present in an amount selected from the group consisting of from about 90% to about 0.001%, from about 90% to about 0.1%, and from about 60% to about 5%, by weight, based on the total dry weight of the candidate compound and surface stabilizer.

28. A method according to any preceding claim, in which the quantity of candidate compound required for the particle size reduction process is selected from the group consisting of less than about 100 mg, less than about 90 mg, less than about 80 mg, less than about 70 mg, less than about 60 mg, less than about 50 mg, less than about 40 mg, less than about 30 mg, less than about 25 mg, less than about 20 mg, less than about 15 mg, less than about 10 mg, less than about 5 mg, less than about 4 mg, less than about 3 mg, less than about 2 mg, and less than about 1 mg.

29. A method according to any preceding claim, in which the total dispersion volume required for the particle size reduction process is selected from the group consisting of less than about 15 mL, less than about 10 mL, less than about 9 mL, less than about 8 mL, less than about 7 mL, less than about 6 mL, less than about 5 mL, less than about 4 mL, less than about 3 mL, and less than about 2 mL.

30. A method according to any preceding claim, in which the time required for the particle size reduction process is selected from the group consisting of about one hour or less, about 45 minutes or less, about 40 minutes or less, about 35 minutes or less, about 30 minutes or less, about 25 minutes or less, about 20 minutes or less, about 15 minutes or less, about 10 minutes or less, and about 5 minutes or less.

31. A method according to any preceding claim, in which the at least one surface stabilizer is present in an amount selected from the group consisting of from about 0.01% to about 90%, from about 1% to about 90%, and from about 5% to about 90%, by weight, based on the total dry weight of the candidate compound and surface stabilizer.

32. A method according to any preceding claim, in which the at least one surface stabilizer is selected from the group consisting of gelatin, casein, lecithin, dextran, gum acacia, cholesterol, tragacanth, stearic acid, benzalkonium chloride, calcium stearate, glycerol monostearate, cetostearyl-alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, polyoxyethylene stearates, colloidal silicon dioxide, phosphates, sodium dodecylsulfate, carboxymethylcellulose calcium, carboxymethylcellulose sodium, methylcellulose, hydroxyethylcellulose, hydroxypropylcellulose, hydroxypropylmethyl-cellulose phthalate, noncrystalline cellulose, magnesium aluminium silicate, tricthanolaminc, polyvinyl alcohol, polyvinylpyrrolidonc, tyloxapol, poloxamers, poloxamines, Tetronic 1508®, dialkylesters of sodium sulfosuccinic acid, sodium lauryl sulfates, alkyl aryl polyether sulfonates, a mixture of sucrose stearate and sucrose distearate, p-isononylphenoxylpoly-(glycidol), Crodestas SL-40®, SA9OHCO which is C₁₈H₃₇CH₂C(O)N(CH₃)-CH₂(CHOH)₄(CH₂OH)₂, decanoyl-N-methylglucamide, n-decyl β-D-glucopyranoside, n-decyl β-D-maltopyranoside, n-dodecyl β-D-glucopyranoside, n-dodecyl β-D-maltoside, heptanoyl-N-methylglucamide, n-heptyl-β-D-glucopyranoside, n-heptyl β-D-thioglucoside, n-hexyl β-D-glucopyranoside, nonanoyl-N-methylglucamide, n-noyl β-D-glucopyranoside, octanoyl-N-methylglucamide, n-octyl-β-D-glucopyranoside, octyl β-D-thioglucopyranoside, PEG-phospholipid, PEG-cholesterol, PEG-cholesterol derivative, PEG-vitamin A, PEG-vitamin E, lysozyme, and random copolymers of vinyl acetate and vinyl pyrrolidone.

33. A method according to any preceding claim, in which the candidate compound is reduced to an effective average particle size selected from the group consisting of less than about 900 run, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, and less than about 50 nm.

34. A method according to any preceding claim, in which at least 60%, 70%, 80%, 90%, or 95% of the candidate compound particles are reduced to a particle size less than the effective average particle size.

## Patentansprüche

1. Hochleistungsdurchmusterungsverfahren, bei dem man
(a) die Teilchengröße von einer oder mehreren Kandidatenverbindungen in einer kleinformatigen Mühle in der Gegenwart von Reibungsmahlmedien reduziert, wobei
(1) die eine oder mehreren Kandidatenverbindungen in einem Flüssigdispersionsmedium gemahlen werden, in welchem die Kandidatenverbindungen schlecht löslich sind,
(2) die gemahlene eine oder die gemahlenen mehreren Kandidatenverbindungen eine effektive durchschnittliche Teilchengröße von weniger als etwa 1 Mikrometer haben und
(3) wenigstens ein Oberflächenstabilisator zu dem Flüssigdispersionsmedium entweder vor oder nach der Teilchengrößenreduzierung in einer Menge hinzugefügt wird, die ausreicht, die effektive durchschnittliche Teilchengröße der einen oder mehreren Kandidatenverbindungen nach der Teilchengrößenreduzierung auf weniger als etwa 1 Mikrometer aufrechtzuerhalten, und
(b) die eine oder mehreren nanopartikulären Kandidatenverbindungen in einem Hochleistungsdurchmusterungstest zur Durchmusterung, ob die eine oder mehreren Verbindungen eine gewünschte Aktivität aufweisen, durchmustert.

2. Hochleistungsdurchmusterungsverfahren, bei dem man
(a) eine oder mehrere Kandidatenverbindungen in einem herkömmlichen Hochleistungsdurchmusterungstest zur Bestimmung, ob die eine oder mehreren Verbindungen eine gewünschte Aktivität aufweisen, durchmustert und
(b) die Teilchengröße der einen oder mehreren Kandidatenverbindungen in einer kleinformatigen Mühle in der Gegenwart von Reibungsmahlmedien reduziert, wobei
(1) die eine oder mehreren Kandidatenverbindungen in einem Flüssigdispersionsmedium gemahlen werden, in welchem die Kandidatenverbindungen schlecht löslich sind,
(2) die gemahlene eine oder die gemahlenen mehreren Kandidatenverbindungen eine effektive durchschnittliche Teilchengröße von weniger als etwa 1 Mikrometer haben und
(3) wenigstens ein Oberflächenstabilisator zu dem Flüssigdispersionsmedium entweder vor oder nach der Teilchengrößenreduzierung in einer Menge hinzugefügt wird, die ausreicht, die effektive durchschnittliche Teilchengröße der einen oder mehreren Kandidatenverbindungen nach der Teilchengrößenreduzierung auf weniger als etwa 1 Mikrometer aufrechtzuerhalten, und
(4) man bestimmt, ob die eine oder mehreren Verbindungen annehmbare Löslichkeit und/oder Dispergierbarkeit aufweisen.

3. Hochleistungsdurchmusterungsverfahren, bei dem man
(a) die Teilchengröße der einen oder mehreren Kandidatenverbindungen unter Anwendung von Homogenisierung reduziert, wobei
(1) die eine oder mehreren Kandidatenverbindungen in ihrer Größe in einem Flüssigdispersionsmedium, in welchem die Kandidatenverbindungen schlecht löslich sind, reduziert werden,
(2) die homogenisierte eine oder die homogenisierten mehreren Kandidatenverbindungen eine effektive durchschnittliche Teilchengröße von weniger als etwa 1 Mikrometer aufweisen und
(3) wenigstens ein Oberflächenstabilisator zu dem Flüssigdispersionsmedium entweder vor oder nach der Teilchengrößenreduzierung in einer Menge hinzugefügt wird, die ausreicht, die effektive durchschnittliche Teilchengröße der einen oder mehreren Kandidatenverbindungen nach der Teilchengrößenreduzierung auf weniger als etwa 1 Mikrometer aufrechtzuerhalten, und
(b) die eine oder mehreren nanopartikulären Kandidatenverbindungen in einem Hochleistungsdurchmusterungstest zur Durchmusterung, ob die eine oder mehreren Verbindungen eine gewünschte Aktivität aufweisen, durchmustert.

4. Hochleistungsdurchmusterungsverfahren, bei dem man
(a) eine oder mehrere Kandidatenverbindungen in einem herkömmlichen Hochleistungsdurchmusterungstest zur Bestimmung, ob die eine oder mehreren Verbindungen eine gewünschte Aktivität aufweisen, durchmustert und
(b) die Teilchengröße der einen oder mehreren Kandidatenverbindungen in einer kleinformatigen Mühle in der Gegenwart von Reibungsmahlmedien reduziert, wobei
(1) die eine oder mehreren Kandidatenverbindungen in einem Flüssigdispersionsmedium gemahlen werden, in welchem die Kandidatenverbindungen schlecht löslich sind,
(2) die gemahlene eine oder die gemahlenen mehreren Kandidatenverbindungen eine effektive durchschnittliche Teilchengröße von weniger als etwa 1 Mikrometer haben und
(3) wenigstens ein Oberflächenstabilisator zu dem Flüssigdispersionsmedium entweder vor oder nach der Teilchengrößenreduzierung in einer Menge hinzugefügt wird, die ausreicht, die effektive durchschnittliche Teilchengröße der einen oder mehreren Kandidatenverbindungen nach der Teilchengrößenreduzierung auf weniger als etwa 1 Mikrometer aufrechtzuerhalten, und
(4) man bestimmt, ob die eine oder mehreren Verbindungen annehmbare Löslichkeit und/oder Dispergierbarkeit aufweisen.

5. Verfahren nach Anspruch 3 oder 4, bei dem die eine oder mehreren Kandidatenverbindungen in der Gegenwart von Reibungsmedien homogenisiert werden.

6. Verfahren nach Anspruch 1, 3 oder 4 oder Anspruch 5, insoweit er von diesen abhängig ist, bei dem die Dispersion von nanopartikulären Kandidatenverbindungen aus Stufe (a) direkt in den Hochleistungsdurchmusterungstest von Stufe (b) eingesetzt wird.

7. Verfahren nach Anspruch 1 oder 3 oder Anspruch 5 oder 6, insoweit sie von diesen abhängig sind, bei dem ein Gemisch von zwei oder mehr Kandidatenverbindungen in Stufe (a) hinsichtlich der Größe reduziert wird.

8. Verfahren nach Anspruch 1 oder 3 oder Anspruch 5, 6 oder 7, insoweit sie von diesen abhängig sind, bei dem ein Gemisch von zwei oder mehr Kandidatenverbindungen in Stufe (b) durchmustert wird.

9. Verfahren nach Anspruch 2 oder 4 oder Anspruch 5 oder 6, insoweit sie von diesen abhängig sind, bei dem ein Gemisch von zwei oder mehr Kandidatenverbindungen in Stufe (b) hinsichtlich der Größe reduziert wird.

10. Verfahren nach Anspruch 2 oder 4 oder Anspruch 5, 6 oder 7, insoweit sie von diesen abhängig sind, bei dem ein Gemisch von zwei oder mehr Kandidatenverbindungen in Stufe (a) durchmustert wird.

11. Verfahren nach einem der vorangegangenen Ansprüche, bei dem das Reibungsmedium oder das Reibungsmahlmedium polymer ist.

12. Verfahren nach einem der vorangegangenen Ansprüche, bei dem das Reibungsmedium oder das Reibungsmahlmedium eine Teilchengröße besitzt, die aus der Gruppe ausgewählt ist, bestehend aus etwa 500 Mikrometer oder weniger, etwa 200 Mikrometer oder weniger, etwa 50 Mikrometer oder weniger und Gemischen davon.

13. Verfahren nach einem der vorangegangenen Ansprüche, bei dem der Hochleistungsdurchmusterungstest ein enzymatischer oder ein Gesamtzellentest ist.

14. Verfahren nach einem der vorangegangenen Ansprüche, bei dem das Dispersionsmedium aus der Gruppe ausgewählt ist, bestehend aus Wasser, wäßrigen Salzlösungen, Distelöl, Ethanol, t-Butanol, 5-Hexan und Glycol.

15. Verfahren nach einem der vorangegangenen Ansprüche, bei dem der Hochleistungsdurchmusterungstest manuell oder automatisch ist.

16. Verfahren nach einem der vorangegangenen Ansprüche, bei dem die Kandidatenverbindung eine Löslichkeit in dem Flüssigdispersionsmedium von weniger als etwa 10 mg/ml besitzt.

17. Verfahren nach einem der vorangegangenen Ansprüche, bei dem die Kandidatenverbindung eine Löslichkeit in dem Flüssigdispersionsmedium von weniger als etwa 1 mg/ml besitzt.

18. Verfahren nach einem der vorangegangenen Ansprüche, bei dem die Kandidatenverbindung mit einem Salz oder einer anderen Substanz konjugiert ist, um die Kandidatenverbindung schlecht löslich zu machen.

19. Verfahren nach einem der vorangegangenen Ansprüche, bei dem die Kandidatenverbindung mit einer Substanz konjugiert ist, die aus der Gruppe ausgewählt ist, bestehend aus hydrophoben Molekülen, Molekülen mit amphipathischen Eigenschaften, Lipidmolekülen, Phospholipidmolekülen, Fetten, Prenylgruppen und Palmitoylgruppen.

20. Verfahren nach Anspruch 18 oder 19, wobei die Konjugation durch ein Verfahren erreicht wird, das aus der Gruppe ausgewählt ist, bestehend aus direkter Konjugation an spezifische Stellen an der Verbindung, Konjugation an den N-terminalen oder C-terminalen Rest der Verbindung über dazwischenliegende Abstandshaltermoleküle und Konjugation über interne Seitenketten an der Verbindung.

21. Verfahren nach einem der vorangegangenen Ansprüche, bei dem die Kandidatenverbindung durch die Hinzufügung von Aminosäureresten entweder während der chemischen Synthese oder der biologischen Expression der Verbindung schlecht löslich gemacht wird.

22. Verfahren nach einem der vorangegangenen Ansprüche, bei dem die Kandidatenverbindung durch Einstellen des pH-Wertes des Dispersionsmediums schlecht löslich gemacht wird.

23. Verfahren nach einem der vorangegangenen Ansprüche, bei dem die Kandidatenverbindung aus der Gruppe ausgewählt ist, bestehend aus einem therapeutischen Mittel, einem Kosmetikum, einem diagnostischen Mittel, einem in der Biotechnologie einsetzbaren Mittel und einem landwirtschaftlichen Mittel.

24. Verfahren nach einem der vorangegangenen Ansprüche, bei dem die Kandidatenverbindung ein landwirtschaftliches Mittel ist, das aus der Gruppe ausgewählt ist, bestehend aus einem Pestizid, einem Düngemittel, einem Insektizid und einem Herbizid.

25. Verfahren nach einem der vorangegangenen Ansprüche, bei dem sich die Zeit zwischen der Durchführung von Stufe (a) und der Durchführung von Stufe (b) bis zu einem Jahr erstreckt.

26. Verfahren nach einem der vorangegangenen Ansprüche, bei dem die Kandidatenverbindung in einer Konzentration zugegen ist, die aus der Gruppe ausgewählt ist, bestehend aus weniger als etwa 50%, weniger als etwa 40%, weniger als etwa 30%, weniger als etwa 25%, weniger als etwa 20%, weniger als etwa 15%, weniger als etwa 10%, weniger als etwa 5%, weniger als etwa 4%, weniger als etwa 3%, weniger als etwa 2%, weniger als etwa 1 %, weniger als etwa 0,5%, weniger als etwa 0,1%, weniger als etwa 0,01% und weniger als etwa 0,001%.

27. Verfahren nach einem der vorangegangenen Ansprüche, bei dem die Kandidatenverbindung in einer Menge zugegen ist, die aus der Gruppe ausgewählt ist, bestehend aus von etwa 90% bis etwa 0,001%, von etwa 90% bis etwa 0,1% und von etwa 60% bis etwa 5%, bezogen auf das Gewicht auf der Grundlage des gesamten Trockengewichts der Kandidatenverbindung und des Oberflächenstabilisators.

28. Verfahren nach einem der vorangegangenen Ansprüche, bei dem die Menge an Kandidatenverbindung, die für das Teilchengrößenreduktionsverfahren erforderlich ist, aus der Gruppe ausgewählt ist, bestehend aus weniger als etwa 100 mg, weniger als etwa 90 mg, weniger als etwa 80 mg, weniger als etwa 70 mg, weniger als etwa 60 mg, weniger als etwa 50 mg, weniger als etwa 40 mg, weniger als etwa 30 mg, weniger als etwa 25 mg, weniger als etwa 20 mg, weniger als etwa 15 mg, weniger als etwa 10 mg, weniger als etwa 5 mg, weniger als etwa 4 mg, weniger als etwa 3 mg, weniger als etwa 2 mg und weniger als etwa 1 mg.

29. Verfahren nach einem der vorangegangenen Ansprüche, bei dem das gesamte Dispersionsvolumen, das für den Teilchengrößenreduktionsprozeß erforderlich ist, aus der Gruppe ausgewählt ist, bestehend aus weniger als etwa 15 ml, weniger als etwa 10 ml, weniger als etwa 9 ml, weniger als etwa 8 ml, weniger als etwa 7 ml, weniger als etwa 6 ml, weniger als etwa 5 ml, weniger als etwa 4 ml, weniger als etwa 3 ml und weniger als etwa 2 ml.

30. Verfahren nach einem der vorangegangenen Ansprüche, bei dem die für den Teilchengrößenreduktionsprozeß erforderliche Zeit aus der Gruppe ausgewählt ist, bestehend aus etwa einer Stunde oder weniger, etwa 45 Minuten oder weniger, etwa 40 Minuten oder weniger, etwa 35 Minuten oder weniger, etwa 30 Minuten oder weniger, etwa 25 Minuten oder weniger, etwa 20 Minuten oder weniger, etwa 15 Minuten oder weniger, etwa 10 Minuten oder weniger und etwa 5 Minuten oder weniger.

31. Verfahren nach einem der vorangegangenen Ansprüche, bei dem der wenigstens eine Oberflächenstabilisator in einer Menge zugegen ist, die aus der Gruppe ausgewählt ist, bestehend aus von etwa 0,01% bis etwa 90%, von etwa 1% bis etwa 90% und von etwa 5% bis etwa 90%, bezogen auf das Gewicht auf der Grundlage des gesamten Trockengewichts der Kandidatenverbindung und des Oberflächenstabilisators.

32. Verfahren nach einem der vorangegangenen Ansprüche, bei dem der wenigstens eine Oberflächenstabilisator aus der Gruppe ausgewählt ist, bestehend aus Gelatine, Kasein, Lecithin, Dextran, Akaziengummi, Cholesterol, Tragant, Stearinsäure, Benzalkoniumchlorid, Calciumstearat, Glycerolmonostearat, Ketostearylalkohol, Ketomakrogol emulgierendem Wachs, Sorbitanestern, Polyoxyethylenalkylestem, Polyoxyethylen-Castoröl-Derivaten, Polyoxyethylen-Sorbitanfettsäureestem, Polyethylenglycolen, Polyoxyethylenstearaten, kolloidalem Siliciumdioxid, Phosphaten, Natriumdodecylsulfat, Carboxymethylzellulose-Calcium, Carboxymethylzellulose-Natrium, Methylzellulose, Hydroxyethyizellulose, Hydroxypropylzellulose, Hydroxypropylmethyl-zellulosephthalat, nicht-kristalliner Zellulose, Magnesiumaluminiumsilikat, Triethanolamin, Polyvinylalkohol, Polyvinylpyrrolidon, Tyloxapol, Poloxameren, Poloxaminen, Tetronic 1508®, Dialkylestem von Natriumsulfosuccinsäure, Natriumlaurylsulfaten, Alkylarylpolyethersulfonaten, einem Gemisch von Sucrosestearat und Sucrosedistearat, p-Isononylphenoxylpolyglycidol, Crodestas SL-40®, SA90HCO, was C₁₈H₃₇CH₂C(O)N(CH₃)-CH₂(CHOH)₄(CH₂OH)₂ ist, Decanoyl-N-methylglucamid, n-Decyl-β-D-glucopyranosid, n-Decyl-β-D-maltopyranosid, n-Dodecyl-β-D-glucopyranosid, n-Dodecyl-β-D-maltosid, Heptanoyl-N-methylglucamid, n-Heptyl-β-D-glucopyranosid, n-Heptyl-β-D-thioglucosid, n-Hexyl-β-D-glucopyranosid, Nonanoyl-N-methylglucamid, n-Nonyl-β-D-glucopyranosid, Octanoyl-N-methylglucamid, n-Octyl-β-D-glucopyranosid, Octyl-β-D-thioglucopyranosid, PEG-Phospholipid, PEG-Cholesterol, PEG-Cholesterolderivat, PEG-Vitamin A, PEG-Vitamin E, Lysozym und zufälligen Copolymeren von Vinylacetat und Vinylpyrrolidon.

33. Verfahren nach einem der vorangegangenen Ansprüche, bei dem die Kandidatenverbindung auf eine effektive durchschnittliche Teilchengröße reduziert wird, die aus der Gruppe ausgewählt ist, bestehend aus weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 150 nm, weniger als etwa 100 nm und weniger als etwa 50 nm.

34. Verfahren nach einem der vorangegangenen Ansprüche, bei dem wenigstens 60%, 70%, 80%, 90% oder 95% der Kandidatenverbindungsteilchen auf eine Teilchengröße von weniger als der effektiven durchschnittlichen Teilchengröße reduziert werden.

## Revendications

1. Procédé de criblage à haut débit comprenant :
(a) une réduction de la taille des particules d'un ou plusieurs composés candidats dans un broyeur à petite échelle en présence d'un milieu de broyage par attrition, où :
(1) le ou les composés candidats sont broyés dans un milieu de dispersion liquide dans lequel les composés candidats sont peu solubles ;
(2) le ou les composés candidats broyés ont une taille moyenne effective de particules inférieure à environ 1 micromètre, et
(3) au moins un stabilisant de surface est ajouté au milieu de dispersion liquide, avant ou après la réduction de taille des particules, en une quantité suffisante pour maintenir la taille moyenne effective de particules du ou des composés candidats, après la réduction de taille des particules, à moins d'environ 1 micromètre ; et
(b) un criblage du ou des composés candidats nanoparticulaires dans un essai de criblage à haut débit classique pour déterminer si le ou les composés candidats ont une activité souhaitée.

2. Procédé de criblage à haut débit comprenant :
(a) un criblage d'un ou plusieurs composés candidats dans un essai de criblage à haut débit classique pour déterminer si le ou les composés ont une activité souhaitée ; et
(b) une réduction de la taille des particules du ou des composés candidats dans un broyeur à petite échelle en présence d'un milieu de broyage par attrition, où :
(1) le ou les composés candidats sont broyés dans un milieu de dispersion liquide dans lequel les composés candidats sont peu solubles ;
(2) le ou les composés candidats broyés ont une taille moyenne effective de particules inférieure à environ 1 micromètre, et
(3) au moins un stabilisant de surface est ajouté au milieu de dispersion liquide, avant ou après la réduction de taille des particules, en une quantité suffisante pour maintenir la taille moyenne effective de particules du ou des composés candidats, après la réduction de taille des particules, à moins d'environ 1 micromètre ; et
(4) déterminer si le ou les composés ont une solubilité et/ou une dispersabilité acceptables.

3. Procédé de criblage à haut débit comprenant :
(a) une réduction de la taille des particules d'un ou plusieurs composés candidats en utilisant une homogénéisation, où :
(1) la réduction de taille du ou des composés candidats est effectuée dans un milieu de dispersion liquide dans lequel les composés candidats sont peu solubles ;
(2) le ou les composés candidats homogénéisés ont une taille moyenne effective de particules inférieure à environ 1 micromètre, et
(3) au moins un stabilisant de surface est ajouté au milieu de dispersion liquide, avant ou après la réduction de taille des particules, en une quantité suffisante pour maintenir la taille moyenne effective de particules du ou des composés candidats, après la réduction de taille des particules, à moins d'environ 1 micromètre ; et
(b) un criblage du ou des composés candidats nanoparticulaires dans un essai de criblage à haut débit classique pour déterminer si le ou les composés candidats ont une activité souhaitée.

4. Procédé de criblage à haut débit comprenant :
(a) un criblage d'un ou plusieurs composés candidats dans un essai de criblage à haut débit classique pour déterminer si le ou les composés ont une activité souhaitée ; et
(b) une réduction de la taille des particules du ou des composés candidats dans un broyeur à petite échelle en présence d'un milieu de broyage par attrition, où :
(1) le ou les composés candidats sont broyés dans un milieu de dispersion liquide dans lequel les composés candidats sont peu solubles ;
(2) le ou les composés candidats broyés ont une taille moyenne effective de particules inférieure à environ 1 micromètre, et
(3) au moins un stabilisant de surface est ajouté au milieu de dispersion liquide, avant ou après la réduction de taille des particules, en une quantité suffisante pour maintenir la taille moyenne effective de particules du ou des composés candidats, après la réduction de taille des particules, à moins d'environ 1 micromètre ; et
(4) déterminer si le ou les composés ont une solubilité et/ou une dispersabilité acceptables.

5. Procédé selon la revendication 3 ou 4, dans lequel le ou les composés candidats sont homogénéisés en présence d'un milieu d'attrition.

6. Procédé selon la revendication 1, 3 ou 4, ou la revendication 5 dans la mesure où elle en dépend, dans lequel la dispersion des composés candidats nanoparticulaires provenant de l'étape (a) est utilisée directement dans l'essai de criblage à haut débit de l'étape (b).

7. Procédé selon la revendication 1 ou 3, ou la revendication 5 ou 6 dans la mesure où elle en dépend, dans lequel un mélange de deux ou plusieurs composés candidats est soumis à une réduction de taille dans l'étape (a).

8. Procédé selon la revendication 1 ou 3, ou la revendication 5, 6 ou 7 dans la mesure où elle en dépend, dans lequel un mélange de deux ou plusieurs composés candidats est criblé dans l'étape (b).

9. Procédé selon la revendication 2 ou 4, ou la revendication 5 ou 6 dans la mesure où elle en dépend, dans lequel un mélange de deux ou plusieurs composés candidats est soumis à une réduction de taille dans l'étape (b).

10. Procédé selon la revendication 2 ou 4, ou la revendication 5, 6 ou 7 dans la mesure où elle en dépend, dans lequel un mélange de deux ou plusieurs composés candidats est criblé dans l'étape (a).

11. Procédé selon l'une quelconque des revendications précédentes, dans lequel le milieu d'attrition ou le milieu de broyage par attrition est polymère.

12. Procédé selon l'une quelconque des revendications précédentes, dans lequel le milieu d'attrition ou le milieu de broyage par attrition a une taille de particules choisie dans le groupe formé par environ 500 micromètres ou moins, environ 200 micromètres ou moins, environ 50 micromètres ou moins, et leurs mélanges.

13. Procédé selon l'une quelconque des revendications précédentes, dans lequel l'essai de criblage à haut débit est un essai enzymatique ou à cellules entières.

14. Procédé selon l'une quelconque des revendications précédentes, dans lequel le milieu de dispersion est choisi dans le groupe formé par l'eau, les solutions aqueuses de sel, l'huile de carthame, l'éthanol, le tertiobutanol, le 5-hexane et le glycol.

15. Procédé selon l'une quelconque des revendications précédentes, dans lequel l'essai de criblage à haut débit est manuel ou automatique.

16. Procédé selon l'une quelconque des revendications précédentes, dans lequel le composé candidat a une solubilité dans le milieu de dispersion liquide inférieure à environ 10 mg/ml.

17. Procédé selon l'une quelconque des revendications précédentes, dans lequel le composé candidat a une solubilité dans le milieu de dispersion liquide inférieure à environ 1 mg/ml.

18. Procédé selon l'une quelconque des revendications précédentes, dans lequel le composé candidat est conjugué à un sel ou une autre substance pour rendre le composé candidat peu soluble.

19. Procédé selon l'une quelconque des revendications précédentes, dans lequel le composé candidat est conjugué à une substance choisie dans le groupe formé par des molécules hydrophobes, des molécules ayant des propriétés amphipathiques, des molécules lipidiques, des molécules phospholipidiques, des graisses, des groupes prényle et des groupes palmitoyle.

20. Procédé selon la revendication 18 ou 19, dans lequel cette conjugaison est effectuée par un procédé choisi dans le groupe formé par la conjugaison directe à des sites spécifiques sur le composé, la conjugaison à un résidu N-terminal ou C-terminal du composé au moyen de molécules d'espacement intermédiaires, et la conjugaison par des chaînes latérales internes sur le composé.

21. Procédé selon l'une quelconque des revendications précédentes, dans lequel le composé candidat est rendu peu soluble par l'addition de résidus d'acides aminés pendant la synthèse chimique ou pendant l'expression biologique du composé.

22. Procédé selon l'une quelconque des revendications précédentes, dans lequel le composé candidat est rendu peu soluble par ajustement du pH du milieu de dispersion.

23. Procédé selon l'une quelconque des revendications précédentes, dans lequel le composé candidat est choisi dans le groupe formé par un agent thérapeutique, un cosmétique, un agent diagnostique, un agent utile en bio-ingénierie et un agent agricole.

24. Procédé selon l'une quelconque des revendications précédentes, dans lequel le composé candidat est un agent agricole choisi dans le groupe formé par un pesticide, un engrais, un insecticide et un herbicide.

25. Procédé selon l'une quelconque des revendications précédentes, dans lequel le temps passé entre l'exécution de l'étape (a) et l'exécution de l'étape (b) s'étend jusqu'à un an.

26. Procédé selon l'une quelconque des revendications précédentes, dans lequel le composé candidat est présent à une concentration choisie dans le groupe formé par moins d'environ 50 %, moins d'environ 40 %, moins d'environ 30 %, moins d'environ 25 %, moins d'environ 20 %, moins d'environ 15 %, moins d'environ 10 %, moins d'environ 5 %, moins d'environ 4 %, moins d'environ 3 %, moins d'environ 2 %, moins d'environ 1 %, moins d'environ 0,5 %, moins d'environ 0,1 %, moins d'environ 0,01 % et moins d'environ 0,001 %.

27. Procédé selon l'une quelconque des revendications précédentes, dans lequel le composé candidat est présent en une quantité choisie dans le groupe formé par environ 90 % à environ 0,001 %, environ 90 % à environ 0,1 %, et environ 60 % à environ 5 %, en poids, par rapport au poids sec total du composé candidat et du stabilisant de surface.

28. Procédé selon l'une quelconque des revendications précédentes, dans lequel la quantité de composé candidat requise pour l'opération de réduction de la taille des particules est choisie dans le groupe formé par moins d'environ 100 mg, moins d'environ 90 mg, moins d'environ 80 mg, moins d'environ 70 mg, moins d'environ 60 mg, moins d'environ 50 mg, moins d'environ 40 mg, moins d'environ 30 mg, moins d'environ 25 mg, moins d'environ 20 mg, moins d'environ 15 mg, moins d'environ 10 mg, moins d'environ 5 mg, moins d'environ 4 mg, moins d'environ 3 mg, moins d'environ 2 mg et moins d'environ 1 mg.

29. Procédé selon l'une quelconque des revendications précédentes, dans lequel le volume total de dispersion requis pour l'opération de réduction de la taille des particules est choisi dans le groupe formé par moins d'environ 15 ml, moins d'environ 10 ml, moins d'environ 9 ml, moins d'environ 8 ml, moins d'environ 7 ml, moins d'environ 6 ml, moins d'environ 5 ml, moins d'environ 4 ml, moins d'environ 3 ml et moins d'environ 2 ml.

30. Procédé selon l'une quelconque des revendications précédentes, dans lequel le temps requis pour l'opération de réduction de la taille des particules est choisi dans le groupe formé par environ une heure ou moins, environ 45 minutes ou moins, environ 40 minutes ou moins, environ 35 minutes ou moins, environ 30 minutes ou moins, environ 25 minutes ou moins, environ 20 minutes ou moins, environ 15 minutes ou moins, environ 10 minutes ou moins et environ 5 minutes ou moins.

31. Procédé selon l'une quelconque des revendications précédentes, dans lequel ledit au moins un stabilisant de surface est présent en une quantité choisie dans le groupe formé par environ 0,01 % à environ 90 %, environ 1 % à environ 90 % et environ 5 % à environ 90 %, en poids, par rapport au poids sec total du composé candidat et du stabilisant de surface.

32. Procédé selon l'une quelconque des revendications précédentes, dans lequel ledit au moins un stabilisant de surface est choisi dans le groupe formé par la gélatine, la caséine, la lécithine, le dextrane, la gomme arabique, le cholestérol, la gomme adragante, l'acide stéarique, le chlorure de benzalkonium, le stéarate de calcium, le monostéarate de glycérol, l'alcool céto-stéarylique, une cire émulsionnante du type cétomacrogol, des esters de sorbitanne, des éthers alkyliques de polyoxyéthylène, des dérivés polyoxyéthyléniques d'huile de ricin, des esters d'acides gras de polyoxyéthylène-sorbitanne, des polyéthylèneglycols, des stéarates de polyoxyéthylène, le dioxyde de silicium colloïdal, des phosphates, le dodécylsulfate de sodium, la carboxyméthylcellulose calcique, la carboxyméthylcellulose sodique, la méthylcellulose, l'hydroxyéthylcellulose, l'hydroxypropylcellulose, le phtalate d'hydroxypropylméthylcellulose, la cellulose non cristalline, le silicate de magnésium et d'aluminium, la triéthanolamine, l'alcool polyvinylique, la polyvinylpyrrolidone, le tyloxapol, les poloxamères, les poloxamines, Tetronic 1508®, des esters dialkyliques d'acide sulfosuccinique sodique, les laurylsulfates de sodium, des alkylarylpolyéthersulfonates, un mélange de stéarate de saccharose et de distéarate de saccharose, le *p*-isononylphénoxypoly(glycidol), Crodestas SL-40®, SA9OHCO qui est C₁₈H₃₇CH₂C (O)N (CH₃)-CH₂(CHOH)₄(CH₂OH)₂, le décanoyl-N-méthylglucamide, le *n*-décyl-β-D-glucopyrannoside, le *n*-décyl-β-D-maltopyrannoside, le *n*-dodécyl-β-D-glucopyrannoside, le *n*-dodécyl-β-D-maltoside, l'heptanoyl-N-méthylglucamide, le *n*-heptyl-β-D-glucopyrannoside, le *n*-heptyl-β-D-thioglucoside, le *n*-hexyl-β-D-glucopyrannoside, le nonanoyl-N-méthylglucamide, le *n*-noyl-β-D-glucopyrannoside, l'octanoyl-N-méthylglucamide, le *n*-octyl-β-D-glucopyrannoside, l'octyl-β-D-thioglucopyrannoside, un PEG-phospholipide, un PEG-cholestérol, un dérivé de PEG-cholestérol, un PEG-vitamine A, un PEG-vitamine E, le lysozyme et des copolymères statistiques d'acétate de vinyle et de vinylpyrrolidone.

33. Procédé selon l'une quelconque des revendications précédentes, dans lequel le composé candidat est réduit à une taille moyenne effective de particules choisie dans le groupe formé par moins d'environ 900 nm, moins d'environ 800 nm, moins d'environ 700 nm, moins d'environ 600 nm, moins d'environ 500 nm, moins d'environ 400 nm, moins d'environ 300 nm, moins d'environ 250 nm, moins d'environ 200 nm, moins d'environ 150 nm, moins d'environ 100 nm et moins d'environ 50 nm.

34. Procédé selon l'une quelconque des revendications précédentes, dans lequel au moins 60 %, 70 %, 80 %, 90 % ou 95 % des particules du composé candidat sont réduites à une taille de particules inférieure à la taille moyenne effective de particules.
